# EUROPÄISCHE PATENTSCHRIFT

(11) **EP 3 380 085 B1**
(45) Veröffentlichungstag und Bekanntmachung des Hinweises auf die Patenterteilung: **13.01.2021**
(21) Anmeldenummer: 15812973.4
(22) Anmeldetag: 27.11.2015
(51) Int. Cl.: A61K 9/46, A61K 9/68, A61K 47/22, A61K 9/00, A61K 31/4439, A61P 1/04, A61K 9/14, A61K 9/20

(54) **ORALE ZUBEREITUNGEN MIT OMEPRAZOL ODER PANTOPRAZOL**
ORAL PREPARATIONS WITH OMEPRAZOLE OR PANTOPRAZOLE
PRÉPARATIONS ORALES CONTENANT DE L'OMÉPRAZOLE OU DU PANTOPRAZOLE

(43) Veröffentlichungstag der Anmeldung: 03.10.2018
(73) Patentinhaber: Symrise AG, 37603 Holzminden (DE)
(72) Erfinder: HANS, Joachim, 37603 Holzminden (DE); LEY, Jakob, 37603 Holzminden (DE); PAETZ, Susanne, 37671 Höxter (DE)
(74) Vertreter: Eisenführ Speiser
(86) Internationale Anmeldenummer: PCT/EP2015/077991
(87) Internationale Veröffentlichungsnummer: WO 2017/088936

(56) Entgegenhaltungen:
- EP-A1- 2 767 829
- WO-A1-03/009846
- WO-A2-2004/066924
- US-A1- 2002 188 019
- LEY J P ET AL: "Evaluation of bitter masking flavanones from Herba Santa (Eriodictyon californicum (H.& A.) Torr., Hydrophyllaceae)", JOURNAL OF AGRICULTURAL AND FOOD CHEMISTRY, AMERICAN CHEMICAL SOCIETY, US, Bd. 53, Nr. 15, 27. Juli 2005 (2005-07-27) , Seiten 6061-6066, XP002696360, ISSN: 0021-8561, DOI: 10.1021/JF0505170 [gefunden am 2005-06-22]

## Beschreibung

### GEBIET DER ERFINDUNG

Die Erfindung befindet sich auf dem Gebiet der pharmazeutischen Wirkstoffe und betrifft orale Zubereitungen mit einem Gehalt an Omeprazol oder Pantoprazol mit verbesserter Galenik.

### STAND DER TECHNIK

Omeprazol und Pantoprazol sind Arzneistoffe aus der Gruppe der Protonenpumpenhemmer, der zur Behandlung von Magen- und Zwölffingerdarmgeschwüren sowie bei Refluxösophagitis eingesetzt wird. Die wichtigsten Indikationen sind:
- Behandlung des Zwölffingerdarmgeschwürs (*Ulcus duodeni*);
- Behandlung des Magengeschwürs (*Ulcus ventriculi*);
- Behandlung der durch Rückfluss von Magensaft verursachten Entzündung der Speiseröhre (Refluxösophagitis);
- Behandlung von Symptomen, die durch den Rückfluss von Magensäure in die Speiseröhre verursacht werden (Refluxkrankheit, Sodbrennen)
- Behandlung des Zollinger-Ellison-Syndroms
- Verhinderung des Wiederauftretens einer Speiseröhrenentzündung oder von Geschwüren des Magens und Zwölffingerdarms, die durch die Einnahme von bestimmten Schmerz- bzw. Rheumamitteln (so genannten nicht-steroidalen Antiphlogistika) bedingt sind, sowie
- Kombinationstherapie von *Helicobacter pylori-*Infektionen*.*

Die Verabreichung von Omeprazol und Pantoprazol erfolgt in der Regel als Tablette oder Kapsel, seltener auch per Infusionslösung.

Von Nachteil ist, dass Omeprazol und Pantoprazol einen intensiven Bittergeschmack aufweisen (betreffend Verfahren zur Identifizierung von Bitterstoffen und bittermodulierenden Stoffen s. z.B. EP 2 767 829 A1), den zu neutralisieren, maskieren oder inhibieren bislang nicht möglich war. Die geschmacklichen Defizite machen die orale Aufnahme unangenehm, was dazu führt, dass Patienten die regelmäßige Einnahme des Medikamentes häufig aussetzen.

Die Aufgabe der vorliegenden Erfindung hat daher darin bestanden, Zubereitungen mit Omeprazol oder Pantoprazol zur Verfügung zu stellen, die frei von den geschilderten Nachteilen sind und sich insbesondere durch einen neutralen Geschmack auszeichnen. Eine weitere Teilaufgabe hat darin bestanden, die Bioverfügbarkeit von Omeprazol und Pantoprazol durch verbesserte Resistenz gegen Magensäure zu verbessern.

### BESCHREIBUNG DER ERFINDUNG

Ein erster Gegenstand der Erfindung betrifft Zubereitungen, enthaltend
(a) Omeprazol und/oder Pantoprazol und
(b) Eriodictyol und Homoeriodictyol.

Überraschenderweise wurde gefunden, dass Eriodictyol und Homoeriodictyol in ausgezeichneter Weise geeignet sind, den Bittergeschmack von Omeprazol oder Pantoprazol zu überdecken bzw. zu maskieren. Zusammen eingesetzt wird die Bitterkeit um etwa die Hälfte reduziert. Es wurde zudem gefunden, dass Mischungen der Komponenten (a) und (b) eine höhere Resistenz gegen Magensäure aufweisen als Omeprazol oder Pantoprazol alleine. Durch Additivieren vom Omeprazol oder Pantoprazol mit einem oder beiden Zusatzstoffen lässt sich somit auch deren Bioverfügbarkeit verbessern.

### OMEPRAZOL

Unter dem Kunstwort Omeprazol wird der Stoff (RS)-5-Methoxy-2-[(4-methoxy-3,5-di-methylpyridin-2-yl)methansulfinyl]-1H-benzoimidazol (I) 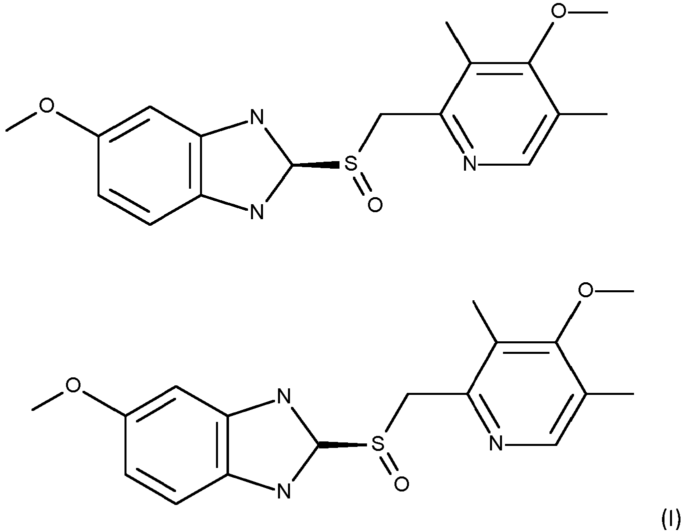 verstanden.

Omeprazol war der erste in die Therapie eingeführte Protonenpumpenhemmer und wurde als gut verträgliches Magenarzneimittel in den 1990er Jahren zugelassen. Seit 1998 wird Omeprazol auch als *Multiple Unit Pellet System* (MUPS) angeboten. Diese Tabletten stellen Komprimate aus kleinen, magensaftresistent überzogenen Pellets dar, die bei Kontakt mit Flüssigkeit rasch zerfallen. Seit dem Jahr 2000 wird das (S)-Enantiomer des Omeprazols (Esomeprazol, Nexium®) vemarktet. Da Esomeprazol vermehrt über CYP2A19 und somit langsamer verstoffwechselt wird als racemisches Omeprazol, resultiert eine verlangsamte Plasmaspiegelabsenkung und somit bessere Bioverfügbarkeit im Vergleich zum Racemat. Jüngste Neuerungen sind Omeprazol-Natriumbikarbonat-Formulierungen (Zegerid®) der US-amerikanischen Firma Santarus. Sie benötigen keinen magensaftresistenten Überzug und ermöglichen zusätzliche Darreichungsformen, die außerdem den Wirkstoff besonders rasch frei setzen sollen. Seit 2004 ist in den USA eine orale Suspension auf dem Markt, 2006 wurden Kapseln zugelassen, mit der Zulassung einer Kautablette ist in Kürze zu rechnen. Soweit die Erfindung auf Omeprazol Bezug nimmt, sind damit alle oben aufgeführten Isomere und galenischen Formen mit umschlossen.

Pantoprazol ((RS)-5-(Difluormethoxy)-2- [(3,4-dimethoxy-2-pyridyl) methylsulfinyl] benzimidazol) (II) ist ebenfalls ein Protonenpumpenhemmer und stellt ein strukturell eng verwandtes Derivat des Omeprazols dar. Es hat die gleichen Indikationen wie Omeprazol und wird in sehr ähnlichen bzw. gleichen Darreichungsformen verabreicht.

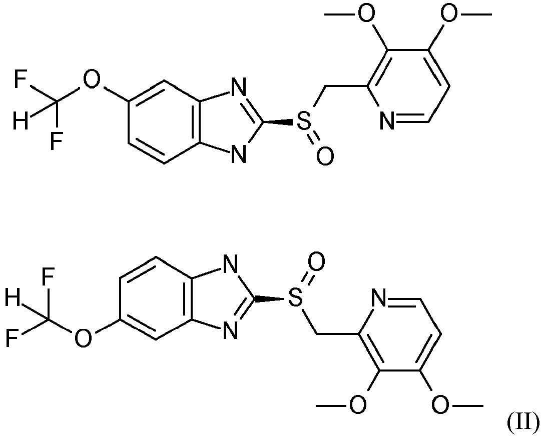

Da Omeprazol und Pantaprazol nur im alkalischen bis neutralem Milieu stabil sind, werden die Wirkstoffe häufig in Tabletten oder Kapseln eingesetzt, die mit einem säureresistenten Überzug versehen sind, so dass eine Auflösung erst im Dünndarm und nicht im sauren Milieu des Dickdarms erfolgt. Alternativ kann man die Stoffe auch auf basische Träger aufziehen, die bei der oralen Aufnahme in alkalisches Mikromilieu erzeugen.

### ERIODICTYOL UND HOMOERIODICTYOL

Eriodictyol 2-(3,4-Dihydroxyphenyl)-5,7-dihydroxy-4-chromanon), und Homoeriodictyol 5-7-Dihydroxy-2-(4-hydroxy-3-methoxyphenyl)-4-chromanon, die in der folgenden Abbildung dargestellt sind, 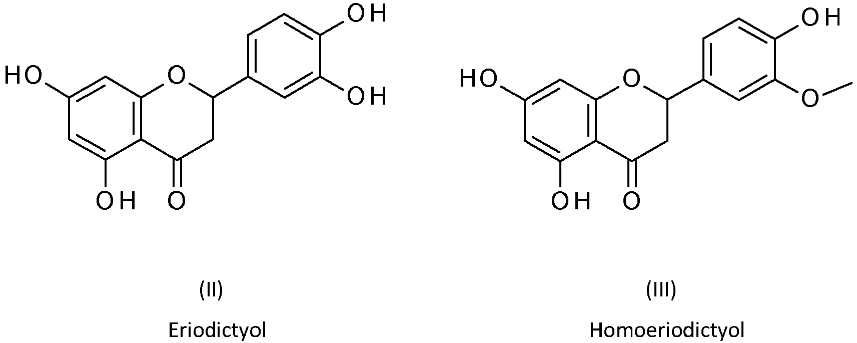 zählen zu den Flavanonen und finden sich als Wirkstoffe beispielsweise in Yerba Santa, *Eriodictyon ssp.* (vgl.). Eriodictyol und Homoeriodictyol werden erfindungsgemäß in Mischungen eingesetzt, wobei die Flavanone als Enantiomerengemisch von 0,1 2S:100 2R bis 0,1 2R:100 2R, als reine Enantiomere, oder bevorzugt als racemisches (50:50) oder nahezu racemisches Gemisch von 35 2S: 65 2R bis 65 2R: 35 2S, besonders bevorzugt 45 2S: 55 2R bis 55 2R: 45 2S eingesetzt werden können. Jedes der beiden Flavanone kann dabei für sich auch ganz oder teilweise in Form eine Salzes vorliegen, bevorzugt ein oder zweiwertige Salze mit Natrium, Kalium, Calcium, Magnesium und Ammonium. Besonders bevorzugt sind dabei Homoeriodictyol im Enantiomerenverhältnis 45 2S: 55 2R bis 55 2R: 45 2S und/oder Homoeriodictyolmono-Natriumsalz im Enantiomerenverhältnis 45 2S: 55 2R bis 55 2R: 45 2S und/oder Eriodictyol im Enantiomerenverhältnis 45 2S: 55 2R bis 55 2R: 45 2S.

### ORALE ZUBEREITUNGEN

Die erfindungsgemäßen oralen Zubereitungen enthalten die Komponenten (a) und (b) vorzugsweise im Gewichtsverhältnis von etwa 4:1 bis 400:1 und insbesondere etwa8:1 bis 200:1. Dabei ist die Komponente (a) Omeprazol bzw. Pantoprazol vorzugsweise in einer Menge von 1.000 bis etwa 30.000 ppm, insbesondere etwa 2.000 bis 20.000 ppm enthalten. Weiterhin bevorzugt ist, dass die oralen Zubereitungen die Komponente (b) Eriodictyol und Homoeriodictyol in einer Menge von etwa 50 bis etwa 500 ppm und insbesondere etwa 100 bis etwa 200 ppm enthält. Bezüglich der Bittermaskierung hat es sich als erfindungsgemäß vorteilhaft erwiesen Eriodictyol und Homoeriodictyol gemeinsam einzusetzen und zwar insbesondere jeweils in einer Menge von etwa 25 bis etwa 250 ppm und bevorzugt etwa 50 bis etwa 150 ppm einzusetzen.

Wie eingangs beschrieben, können die oralen Darreichungsformen von Omeprazol oder Pantoprazol flüssig vorliegen. Die bevorzugte galenischen Formen sind jedoch orale Darreichungsformen, insbesondere orale fest oder flüssige Darreichungsformen und können z.B. in Form von Lösungen, Suspensionen, Emulsionen, Pulver zum Schlucken, Kautabletten, Komprimattabletten, Kapseln, lackierte Tabletten, Lutschbonbons, Kaubonbons, Fruchtgummis oder medizinische Kaugummis umfassen. Sofern die Ausführungsformen fest sind, weisen sie wie oben erläutert vorzugsweise einen säureresistenten Überzug (bei Tabletten) oder Hülle (bei Kapseln) auf.

### TABLETTEN

Bei den erfindungsgemäßen oralen Zubereitungen kann es sich auch um Tabletten handeln. Tabletten können die für diese Darreichungsform weiteren üblichen Bestandteile enthalten, als da beispielsweise sind: Trägerstoffe, Sprengmittel und/oder Farb- und Aromastoffe. Die Tablettierung selbst stellt ein hinlänglich bekanntes großtechnisches Verfahren dar. Exemplarisch sei auf die DE 102006051529 A1 (HENKEL) verwiesen, in der die Herstellung von Tabletten ausführlich beschrieben wird.

### Trägerstoffe

Geeignete Trägerstoffe lassen sich in Füll- und Bindemittel untergliedern. Füllmittel sorgen dafür, dass die Tablette die notwendige Größe/Masse erhält. Eingesetzt werden Stärken (Mais-, Kartoffel- und Weizenstärke) und Lactose. Weitere Füllmittel sind: Glucose, Mannitol, Sorbitol. Fructose wird auf Grund ihres hohen Preises nur sehr selten verwendet. Saccharose wird vor allem für Lutschtabletten verwendet.

Bindemittel sorgen hingegen für den Zusammenhalt in den Granulaten oder Pulvern und neben dem Pressdruck für die Festigkeit von Tabletten. Sie unterteilen sich in Trockenbindemittel wie z. B. MCC (mikrokristalline Cellulose) oder Stärke und in Feuchtbindemittel/Klebstoffe für die Granulierung wie z. B. Stärkekleister, Celluloseether, Kollidon und Gelatine.

### Sprengmittel

Um den Zerfall vorgefertigter Formkörper zu erleichtern, ist es möglich, Desintegrationshilfsmittel, sogenannte Tablettensprengmittel, in diese Mittel einzuarbeiten, um die Zerfallszeiten zu verkürzen. Unter Tablettensprengmitteln bzw. Zerfallsbeschleunigern werden Hilfsstoffe verstanden, die für den raschen Zerfall von Tabletten in Wasser oder anderen Medien und für die zügige Freisetzung der Wirkstoffe sorgen.

Diese Stoffe, die auch aufgrund ihrer Wirkung als "Spreng" mittelbezeichnet werden, vergrößern bei Wasserzutritt ihr Volumen, wobei einerseits das Eigenvolumen vergrößert (Quellung), andererseits auch über die Freisetzung von Gasen ein Druck erzeugt werden kann, der die Tablette in kleinere Partikel zerfallen lässt. Altbekannte Desintegrationshilfsmittel sind beispielsweise Carbonat/Zitronensäure-Systeme, wobei auch andere organische Säuren eingesetzt werden können. Quellende Desintegrationshilfsmittel sind beispielsweise synthetische Polymere wie Polyvinylpyrrolidon (PVP) oder natürliche Polymere bzw. modifizierte Naturstoffe wie Cellulose und Stärke und ihre Derivate, Alginate oder Casein-Derivate.

Als bevorzugte Desintegrationsmittel werden Desintegrationsmittel auf Cellulosebasis eingesetzt. Reine Cellulose weist die formale Bruttozusammensetzung (C₆H₁₀O₅)n auf und stellt formal betrachtet ein beta-1,4-Polyacetat von Cellobiose dar, die ihrerseits aus zwei Molekülen Glucose aufgebaut ist. Geeignete Cellulosen bestehen dabei aus ca. 500 bis 5000 Glucose-Einheiten und haben demzufolge durchschnittliche Molmassen von 50.000 bis 500.000. Als Desintegrationsmittel auf Cellulosebasis verwendbar sind im Rahmen der vorliegenden Erfindung auch Cellulose-Derivate, die durch polymer analoge Reaktionen aus Cellulose erhältlich sind. Solche chemisch modifizierten Cellulosen umfassen dabei beispielsweise Produkte aus Veresterungen bzw. Veretherungen, in denen Hydroxygruppen durch Wasserstoffatome substituiert wurden. Aber auch Cellulosen, in denen die Hydroxy-Gruppen gegen funktionelle Gruppen, die nicht über ein Sauerstoffatom gebunden sind, ersetzt wurden, lassen sich als Cellulose-Derivate einsetzen. In die Gruppe der Cellulose-Derivate fallen beispielsweise Alkalicellulosen, Carboxymethylcellulose (CMC), Celluloseester und -ether sowie Aminocellulosen. Die genannten Cellulosederivate werden vorzugsweise nicht allein als Desintegrationsmittel auf Cellulosebasis eingesetzt, sondern in Mischung mit Cellulose verwendet. Der Gehalt dieser Mischungen an Cellulosederivaten beträgt vorzugsweise unterhalb 50 Gew.- percent, besonders bevorzugt unterhalb 20Gew.- %, bezogen auf das Desintegrationsmittel auf Cellulosebasis. Besonders bevorzugt wird als Desintegrationsmittel auf Cellulosebasis reine Cellulose eingesetzt, die frei von Cellulosederivaten ist.

Die als Sprengmittel eingesetzte Cellulose wird vorzugsweise nicht in feinteiliger Form eingesetzt, sondern vor dem Zumischen zu den zu verpressenden Vorgemischen in eine gröbere Form überführt, beispielsweise granuliert oder kompaktiert. Die Teilchengrößen solcher Desintegrationsmittel liegen zumeist oberhalb 200 µm vorzugsweise zu mindestens 90 Gew.-% zwischen 300 und 1.600 µm und insbesondere zu mindestens 90 Gew.-% zwischen 400 und 1.200 µm.

Als weiteres Sprengmittel auf Cellulosebasis oder als Bestandteil dieser Komponente kann mikrokristalline Cellulose eingesetzt werden. Diese mikrokristalline Cellulose wird durch partielle Hydrolyse von Cellulosen unter solchen Bedingungenerhalten, die nur die amorphen Bereiche (ca. 30 % der Gesamt-Cellulosemasse) der Cellulosen angreifen und vollständig auflösen, die kristallinen Bereiche (ca. 70%) aber unbeschadet lassen. Eine nachfolgende Desaggregation der durch die Hydrolyse entstehenden mikrofeinen Cellulosen liefert die mikrokristallinen Cellulosen, die Primärteilchengrößen von ca. 5 µm aufweisen und beispielsweise zu Granulaten mit einer mittleren Teilchengröße von 200 µm kompaktierbar sind.

Erfindungsgemäß bevorzugt können darüber hinaus weiterhin gasentwickelnde Brausesysteme eingesetzt werden. Das gasentwickelnde Brausesystem kann aus einer einzigen Substanzbestehen, die bei Kontakt mit Wasser ein Gas freisetzt. Unter diesen Verbindungen ist insbesondere das Magnesiumperoxid zu nennen, das bei Kontakt mit Wasser Sauerstofffreisetzt. Üblicherweise besteht das gasfreisetzende Sprudelsystem jedoch seinerseits aus mindestens zwei Bestandteilen, die miteinander unter Gasbildung reagieren. Während hier eine Vielzahl von Systemen denk- und ausführbar ist, die beispielsweise Stickstoff, Sauerstoff oder Wasserstoff freisetzen, wird sich das in den Wasch-und Reinigungsmittel eingesetzte Sprudelsystem sowohl anhand ökonomischer als auch anhand ökologischer Gesichtspunkte auswählen lassen. Bevorzugte Brausesysteme bestehen aus Alkalimetallcarbonat und/oder - hydrogencarbonat sowie einem Acidifizierungsmittel, das geeignet ist, aus den Alkalimetallsalzen in wässriger Lösung Kohlendioxid freizusetzen.

### Farbstoffe

Lebensmittelfarbstoffe oder kurz Farbstoffe sind Lebensmittelzusatzstoffe zum Färben von Zubereitungen, die zum Verzehr geeignet sind. Farbstoffe werden in die Gruppen der natürlichen Farbstoffe und synthetischen Farbstoffe unterteilt. Die naturidentischen Farbstoffe sind ebenfalls synthetischen Ursprungs. Die naturidentischen Farbstoffe sind synthetische Nachbildungen von in der Natur vorkommenden, färbenden Substanzen. Geeignete Farbstoffe für den Einsatz in der vorliegenden Zusammensetzung sind ausgewählt aus: Kurkumin, E 100 Riboflavin, Lactoflavin, Laktoflavin, Vitamin B2, E 101 Tartrazin, E 102 Chinolingelb, E 104 Gelborange S, Gelborange RGL, E 110 Cochenille, Karminsäure, echtes Karmin, E 120 Azorubin, Carmoisin, E 122 Amaranth, E 123 Cochenillerot A, Ponceau 4 R, Victoriascharlach 4 R, E 124 Erythrosin, E 127 Allurarot AC, E 129 Patentblau V, E 131 Indigotin, Indigo-Karmin, E 132 Brillantblau FCF, Patentblau AE, Amidoblau AE, E 133 Chlorophylle, Chlorophylline, E 140 Kupferkomplexe der Chlorophylle, Kupfer-Chlorophyllin-Komple, E 141 Brillantsäuregrün, Grün S, E 142 Zuckerkulör, Zuckercouleur, E 150 a Sulfitlaugen-Zuckerkulör, E 150 b Ammoniak-Zuckerkulör, E 150 c Ammoniumsulfit-Zuckerkulör, E 150 d Brillantschwarz FCF, Brillantschwarz PN, Schwarz PN, E 151 Pflanzenkohle, E 153 Braun FK, E 154 Braun HT, E 155 Carotin, Karotin, E 160 a Annatto, Bixin, Norbixin, E 160 b Capsanthin, Capsorubin, E 160 c Lycopin, E 160 d Beta-apo-8'-Carotinal, Apocarotinal, Beta-Apocarotinal, E 160 e Beta-apo-8'-Carotin-säure-Ethylester (C30), Apocarotinester, Beta-Carotinsäureester, E 160 f Lutein, Xanthophyll, E 161 b Canthaxanthin, E 161 g Betanin, Betenrot, E 162 Anthocyane, E 163 Calciumcarbonat, E 170 Titandioxid, E 171 Eisenoxide, Eisenhydroxide, E 172 Aluminium, E 173 Silber, E 174 Gold, E 175 Litholrubin BK, Rubinpigment BK, E 180.

### Aromastoffe

Die erfindungsgemäßen Zubereitungen können einen oder mehrere Aromastoffe enthalten; Aromastoffe sind definiert durch z.B. europäische oder amerikanische Rechtssprechung, z.B. die Verordnung (EG) Nr. 2232/96 des Europäischen Parlaments und des Rates und der Positivliste, die in der Durchführungsverordnung (EU) Nr. 872/2012 der Kommission definiert ist. Typische Beispiele umfassen: Acetophenon, Allylcapronat, alpha-lonon, beta-lonon, Anisaldehyd, Anisylacetat, Anisylformiat, Benzaldehyd, Benzothiazol, Benzylacetat, Benzylalkohol, Benzylbenzoat, beta-lonon, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, Decalacton, Dihydrocumarin, Dimethylanthranilat, Dimethylanthranilat, Dodecalacton, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, 4-(p-Hydroxyphenyl)-2-butanon, gamma-Decalacton, Geraniol, Geranylacetat, Geranylacetat, Grapefruitaldehyd, Methyldihydrojasmonat (z.B. Hedion®), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-2-Hexenylacetat, cis-3-Hexenylacetat, cis-3-Hexenylcapronat, trans-2-Hexenylcapronat, cis-3-Hexenylformiat, cis-2-Hexylacetat, cis-3-Hexylacetat, trans-2-Hexylacetat, cis-3-Hexylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyldihydrojasmonat, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, 2,4-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylalkohol, Phenylethylisovalerianat, Piperonal, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4-isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-1-al und Phenylacetaldehyd, 2-Methyl-3-(methylthio)furan, 2-Methyl-3-furanthiol, bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-l-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-lsopropyl-2-methoxypyrazin, 3-lsobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, 4-Hydroxy-2,5-dimethyl-3(2H)-furanon, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie (hier nicht explizit genannte) Stereoisomere, Enantiomere, Stellungsisomere, Diastereomere, cis/trans-Isomere bzw. Epimere dieser Substanzen.

Aromastoffe können auch den Geschmack direkt beeinflussen (Geschmacksmodulatoren) und sind beispielsweise ausgewählt aus der Gruppe bestehend aus Mononatriumglutamat, freie Glutaminsäure, Nucleotide oder deren pharmazeutisch akzeptable Salze, Strombine, Theogalline wie in JP 2007 110988 A beschrieben, Pyridin-Betain-Verbindungen wie in EP 1291342 B1 beschrieben, Glutaminsäureglycoside wie in WO 2002 087361 A1 beschrieben, Äpfelsäureglycoside wie in WO 2006 003107 A1 beschrieben, Glutathion-Derivate wie in EP 0181421 oder WO 2007 042273 A1 beschrieben), Alkylpyridine (vorzugsweise Alkylpyridine wie in WO 2009 122318 A1 und WO 2009 1223319 A1 beschrieben), insbesondere 2-Hexyl-, 2-Heptyl und 2-Octylpyridin, (2E,6Z)-N-cyclopropylnona-2,6-dienamid, (2E,6Z)-N-ethylnona-2,6-dienamid, N-[(2E)-3,7-dimethylocta-2,6-dienyl]cyclopro-pancarboxamid, N'-[(2-methoxy-4-methyl-phenyl)methyl]-N-[2-(5-methyl-2-pyridyl)ethyl]-oxa-mid, N'-[(2,4-dimethoxyphenyl)methyl]-N-[2-(2-pyridyl)ethyl]oxamid, N'-[(2-methoxy-4-methyl-phenyl)methyl]-N-[2-(2-pyridyl)ethyl]oxamid, N-(1-propylbutyl)-1,3-benzodioxole-5-carb-oxamid, 1-(2-hydroxy-4-isobutoxy-phenyl)-3-(2-pyridyl)propan-1-on und 1-(2-hydroxy-4-methoxy-phenyl)-3-(2-pyridyl)propan-1-on, Zimtsäureamide wie in EP 2,529,632-B1 oder WO 2013 000,673 beschrieben), Lactisole, Hesperitin nach EP 1909599 A1, Phloretin nach EP 1972203 B1 oder EP 1998636 B1, Hydroxyflavane nach US 2010 292175 AA, 4-Hydroxychalcone nach EP 1972203 B1, Extrakte auf Basis von Hydrangea dulcis gemäß EP 2298084 A2, oder Rubus ssp.; Gemische von Molkeproteinen mit Lecithinen, Hefeextrakte, Pflanzenhydrolysate, pulverisierte Gemüse (z.B. Zwiebelpulver, Tomatenpulver), Pflanzenextrakte (z.B. von Liebstöckel oder von Pilzen wie Shiitake), Meeralgen und Mineralsalzmischungen, insbesondere Mineralsalzmischungen nach US 2009 214728 AA, Rubemamin oder Rubescenamin nach EP 2,529,632-B1.

### KAUGUMMIS

Bei den erfindungsgemäßen oralen Zubereitungen kann es sich auch um Kaugummis handeln. Diese Produkte enthalten typischerweise eine wasserunlösliche und eine wasserlösliche Komponente.

Die wasserunlösliche Basis, die auch als "Gummibasis" bezeichnet wird. umfasst üblicherweise natürliche oder synthetische Elastomere, Harze, Fette und Öle, Weichmacher, Füllstoffe, Farbstoffe sowie gegebenenfalls Wachse. Der Anteil der Basis an der Gesamtzusammensetzung macht üblicherweise 5 bis 95, vorzugsweise 10 bis 50 und insbesondere 20 bis 35 Gew.-% aus. In einer typischen Ausgestaltungsform der Erfindung setzt sich die Basis aus 20 bis 60 Gew.-% synthetischen Elastomeren, 0 bis 30 Gew.-% natürlichen Elastomeren, 5 bis 55 Gew.-% Weichmachern, 4 bis 35 Gew.-% Füllstoffe und in untergeordneten Mengen Zusatzstoffe wie Farbstoffe, Antioxidantien und dergleichen zusammen, mit der Maßgabe, dass sie allenfalls in geringen Mengen wasserlöslich sind.

Als geeignete synthetische Elastomere kommen beispielsweise Polyisobutylene mit durchschnittlichen Molekulargewichten (nach GPC) von 10.000 bis 100.000 und vorzugsweise 50.000 bis 80.000, Isobutylen-Isopren-Copolymere ("Butyl Elastomere"), Styrol-Butadien-Copolymere (Styrol:Butadien-Verhältnis z.B. 1: 3 bis 3: 1), Polyvinylacetate mit durchschnittlichen Molekulargewichten (nach GPC) von 2.000 bis 90.000 und vorzugsweise 10.000 bis 65.000, Polyisoprene, Polyethylen, Vinylacetat-Vinyllaurat-Copolymere und deren Gemische. Beispiele für geeignete natürliche Elastomere sind Kautschuks wie etwa geräucherter oder flüssiger Latex oder Guayule sowie natürliche Gummistoffe wie Jelutong, Lechi caspi, Perillo, Sorva, Massaranduba balata, Massaranduba chocolate, Nispero, Rosindinba, Chicle, Gutta hang lkang sowie deren Gemische. Die Auswahl der synthetischen und natürlichen Elastomere und deren Mischungsverhältnisse richtet sich im Wesentlichen danach, ob mit den Kaugummis Blasen erzeugt werden sollen ("bubble gums") oder nicht. Vorzugsweise werden Elastomergemische eingesetzt, die Jelutong, Chicle, Sorva und Massaranduba enthalten.

In den meisten Fällen erweisen sich die Elastomere in der Verarbeitung als zu hart oder zu wenig verformbar, so dass es sich als vorteilhaft erwiesen hat, spezielle Weichmacher mitzuverwenden, die natürlich insbesondere auch alle Anforderungen an die Zulassung als Nahrungsmittelzusatzstoffe erfüllen müssen. In dieser Hinsicht kommen vor allem Ester von Harzsäuren in Betracht, beispielsweise Ester von niederen aliphatischen Alkoholen oder Polyolen mit ganz oder teilweise gehärteten, monomeren oder oligomeren Harzsäuren. Insbesondere werden für diesen Zweck die Methyl-, Glycerin-, oder Pentareythritester sowie deren Gemische eingesetzt. Alternativ kommen auch Terpenharze in Betracht, die sich von alpha-Pinen, beta-Pinen, delta-Limonen oder deren Gemischen ableiten können.

Als Füllstoffe oder Texturiermittel kommen Magnesium- oder Calciumcarbonat, gemahlener Bimsstein, Silicate, speziell Magnesium- oder Aluminiumsilicate, Tone, Aluminiumoxide. Talkum, Titandioxid, Mono-, Di- und Tricalciumphosphat sowie Cellulosepolymere.

Geeignete Emulgatoren sind Talg, gehärteter Talg, gehärtete oder teilweise gehärtete pflanzliche Öle, Kakaobutter, Partialglyceride, Lecithin, Triacetin und gesättigte oder ungesättigte Fettsäuren mit 6 bis 22 und vorzugsweise 12 bis 18 Kohlenstoffatomen sowie deren Gemische.

Als Farbstoffe und Weißungsmittel kommen beispielsweise die für die Färbung von Lebensmitteln zugelassenen FD und C-Typen, Pflanzen- und Fruchtextrakte sowie Titandioxid in Frage.

Die Basismassen können Wachse enthalten oder wachsfrei sein; Beispiele für wachsfreie Zusammensetzungen finden sich unter anderem in der Patentschrift US 5,286,500**,** auf deren Inhalt hiermit ausdrücklich Bezug genommen wird. Zusätzlich zu der wasserunlöslichen Gummibasis enthalten Kaugummizubereitungen regelmäßig einen wasserlösliche Anteil, der beispielsweise von Weichmacher, Süßstoffen, Füllstoffen, Geschmacksstoffen, Geschmacksverstärkern, Emulgatoren, Farbstoffen, Säuerungsmitteln, Antioxidantien und dergleichen gebildet werden, hier mit der Maßgabe, dass die Bestandteile eine wenigstens hinreichende Wasserlöslichkeit besitzen. In Abhängigkeit der Wasserlöslichkeit der speziellen Vertreter können demnach einzelne Bestandteile sowohl der wasserunlöslichen wie auch der wasserlöslichen Phase angehören. Es ist jedoch auch möglich, Kombinationen beispielsweise eines wasserlöslichen und eines wasserunlöslichen Emulgators einzusetzen, wobei sich die einzelnen Vertreter, dann in unterschiedlichen Phasen befinden. Üblicherweise macht der wasserunlösliche Anteil 5 bis 95 und vorzugsweise 20 bis 80 Gew.-% der Zubereitung aus.

Wasserlösliche Weichmacher werden den Kaugummizubereitungen hinzugegeben um die Kaubarkeit und das Kaugefühl zu verbessern und sind in den Mischungen typischerweise in Mengen von 0,5 bis 15 Gew.-% zugegen. Typische Beispiele sind Glycerin, Lecithin sowie wässrige Lösungen von Sorbitol, gehärteten Stärkehydrolysaten oder Kornsirup.

Als Süßstoffe kommen sowohl zuckerhaltige wie zuckerfreie Verbindungen in Frage, die in Mengen von 5 bis 95, vorzugsweise 20 bis 80 und insbesondere 30 bis 60 Gew.-% bezogen auf die Kaugummizusammensetzung eingesetzt werden. Typische Saccharid-Süssstoffe sind Sucrose, Dextrose, Maltose, Dextrin, getrockneter Invertzucker, Fructose, Levulose, Galactose, Kornsirup sowie deren Gemische. Als Zuckerersatzstoffe kommen Sorbitol, Mannitol, Xylitol, gehärtete Stärkehydrolysate, Maltitol und deren Gemische in Frage. Weiterhin kommen als Zusatzstoffe auch sogenannte HIAS ("High Intensity Articifical Sweeteners") in Betracht, wie beispielsweise Sucralose, Aspartam, Acesulfamsalze, Alitam, Saccharin und Saccharinsalze, Cyclamsäure und deren Salze, Glycyrrhizine, Dihydrochalcone, Thaumatin, Monellin und dergleichen alleine oder in Abmischungen. Besonders wirksam sind auch die hydrophoben HIAS, die Gegenstand der internationalen Patentanmeldung WO 2002 091849 A1 (Wrigleys) sowie Stevia Extrakte und deren aktiven Bestandteile, insbesondere Rebaudiosid A sind. Die Einsatzmenge dieser Stoffe hängt in erster Linie von ihrem Leistungsvermögen ab und liegt typischerweise im Bereich von 0,02 bis 8 Gew.-%.

Insbesondere für die Herstellung kalorienarmer Kaugummis eignen sich Füllstoffe wie beispielsweise Polydextrose, Raftilose, Rafitilin, Fructooligosaccharide (NutraFlora), Palatinoseoligosaaccharide, Guar Gum Hydrolysate (Sun Fiber) sowie Dextrine.

Die Auswahl an weiteren Geschmacksstoffen ist praktisch unbegrenzt und für das Wesen der Erfindung unkritisch. Üblicherweise liegt der Gesamtanteil aller Geschmacksstoffe bei 0,1 bis 15 und vorzugsweise 0,2 bis 5 gew.-% bezogen auf die Kaugummizusammensetzung. Geeignete weitere Geschmacksstoffe stellen beispielsweise essentielle Öle, synthetische Aromen und dergleichen dar, wie etwa Anisöl, Sternanisöl, Kümmelöl, Eukalyptusöl, Fenchelöl, Citronenöl, Wintergrünöl, Nelkenöl, und dergleichen, wie sie auch beispielsweise in Mund-und Zahnpflegemittel Verwendung finden.

### KAPSELN

Unter Kapseln, die die erfindungsgemäßen Zubereitungen enthalten können, sind sphärische Aggregate zu verstehen, die mindestens einen festen oder flüssigen Kern enthalten, der von mindestens einer kontinuierlichen Hülle umschlossen ist. Die Wirkstoffe können durch Überzugsmaterialien verkapselt werden und dabei als Makrokapseln mit Durchmessern von etwa 0,5 bis etwa 15 mm oder Mikrokapseln mit Durchmessern von etwa 0,0001 bis etwa 0,5 mm vorliegen.

### Überzugsmaterialien

Geeignete Überzugsmaterialien sind dabei beispielsweise Stärken, einschließlich deren Abbauprodukten sowie chemisch oder physikalisch erzeugten Derivaten (insbesondere Dextrine und Maltodextrine), Gelatine, Gummi Arabicum, Agar-Agar, Ghatti Gum, Gellan Gum, modifizierte und nicht-modifizierte Cellulosen, Pullulan, Curdlan, Carrageenane, Alginsäure, Alginate, Pektin, Inulin, Xanthan Gum und Mischungen von zwei oder mehreren dieser Substanzen.

Das feste Verkapselungsmaterial ist vorzugsweise eine Gelatine (insbesondere Schweine-, Rind-, Geflügel- und/oder Fischgelatine), wobei diese vorzugsweise einen Schwellfaktor von größer oder gleich 20, vorzugsweise von größer oder gleich 24 aufweist. Unter diesen Stoffen ist Gelatine besonders bevorzugt, da sie gut verfügbar ist und mit unterschiedlichen Schwellfaktoren bezogen werden kann.

Ebenfalls bevorzugt sind Maltodextrine (insbesondere auf Basis von Getreide, speziell Mais, Weizen, Tapioka oder Kartoffeln), die vorzugsweise DE-Werte im Bereich von 10 bis 20 aufweisen. Weiterhin bevorzugt sind Cellulosen (z.B. Celluloseether), Alginate (z.B. Natriumalginat), Carrageenan (z.B. beta-, jota-, lambda- und/oder kappa-Carrageenan), Gummi Arabicum, Curdlan und/oder Agar Agar.

Ebenfalls bevorzugt sind Alginatkapseln wie sie beispielsweise in den folgenden Schriften ausführlich beschrieben werden: EP 0389700 A1**,** US 4,251,195**,** US 6,214,376**,** WO 2003 055587 oder WO 2004 050069 A1**.**

In einer weiteren bevorzugten Ausführungsform besteht die Hülle der Kapseln aus Koazervationsprodukten aus kationischen Monomeren oder Biopolymeren (wie z.B. Chitosan) und anionischen Monomeren, wie beispielsweise (Meth)Acrylaten oder Alginaten.

### Verkapselungsverfahren

Bei den Kapseln handelt es sich im Allgemeinen um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Nach einem dritten Verfahren werden Partikel abwechselnd mit Polyelektrolyten unterschiedlicher Ladung beschichtet ("layer-by-layer"-Verfahren). Die mikroskopisch kleinen Kapseln lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide).

Chitosanmikrokapseln und Verfahren zu ihrer Herstellung sind aus dem Stand der Technik hinlänglich bekannt **[**WO 01/01926**,** WO 01/01927**,** WO 01/01928**,** WO 01/01929**].** Mikrokapseln mit mittleren Durchmessern im Bereich von 0,0001 bis 5, vorzugsweise 0,001 bis 0,5 und insbesondere 0,005 bis 0,1 mm, bestehend aus einer Hüllmembran und einer die Wirkstoffe enthaltenden Matrix, können beispielsweise erhalten werden, indem man
(a) aus Gelbildnern, kationischen Polymeren und Wirkstoffen eine Matrix zubereitet,
(b) gegebenenfalls die Matrix in einer Ölphase dispergiert,
(c) die dispergierte Matrix mit wässrigen Lösungen anionischer Polymere behandelt und gegebenenfalls dabei die Ölphase entfernt.

Die Schritte (a) und (c) sind dabei insofern austauschbar, als man anstelle der kationischen Polymeren in Schritt (a) anionische Polymere einsetzt und umgekehrt.

Man kann die Kapseln auch erzeugen, indem man den Wirkstoff abwechselnd mit Schichten aus unterschiedlich geladenen Polyelektrolyten einhüllt (layer-by-layer-Technologie). In diesem Zusammenhang sei auf das Europäische Patent EP 1064088 B1 (Max-Planck Gesellschaft) verwiesen.

### ARZNEIMITTEL

Ein weiterer Gegenstand der vorliegenden Erfindung betrifft Zubereitungen, enthaltend
(a) Omeprazol oder Pantoprazol
(b) Eriodictyol und Homoeriodictyol
als Arzneimittel, speziell zur Verwendung als Arzneimittel zur Behandlung von Entzündungszuständen des Magens und des Darms sowie Refluxösophagitis. Insbesondere sind diese Mittel zur oralen Aufnahme bestimmt. Bei diesen Mitteln kann es sich um Tabletten, Kapseln, Kautabletten, Lösungen, Emulsionen, Suspensionen, Pulver und Granulate zur direkten Einnahme, Pulver zum Auflösen in Wasser, Kaubonbons, Lutschbonbons, Gummibonbons, Kaugummis, Fruchtgummis und dergleichen handeln.

### GEWERBLICHE ANWENDBARKEIT

Zwei weitere Gegenstände der Erfindung betreffen zum einen ein Verfahren zur Maskierung des Bittergeschmacks von pharmazeutischen Zubereitungen mit einem Gehalt an Omeprazol, umfassend die folgenden Schritte:
(i) Bereitstellen einer Trägermasse zur Herstellung einer pharmazeutischen Zubereitung;
(ii) Versetzen der Masse aus Schritt (i) mit einer Menge an Omeprazol oder Pantoprazol;
(iii) Versetzen der Masse aus Schritt (ii) mit einer Menge an Eriodictyol und Homoeriodictyol; und
(iv) Verarbeiten der Masse aus Schritt (iii) zu einem pharmazeutischen Endprodukt,
sowie zum anderen die Verwendung von Eriodictyol und Homoeriodictyol zur Maskierung oder Inhibierung des Bittergeschmacks von Omeprazol oder Pantoprazol;.

Bei dem pharmazeutischen Endprodukt kann es sich beispielsweise um eine Tablette, ein Lutsch- oder Kaubonbon, ein Kaugummi, ein Fruchtgummi oder eine Kapsel handeln.

Insofern vorstehend bevorzugte Ausführungsformen, Stoffmischungen und Mengenangaben genannt worden sind, treffen diese für das beanspruchte Verfahren bzw. für die beanspruchte Verwendung ebenso zu, so dass sich eine Wiederholung erübrigt.

### BEISPIELE

### BEISPIELE 1 BIS 3, VERGLEICHSBEISPIELE V1 und V2

Um den Bittermaskierungeffekt zu prüfen, wurden wässrige Lösungen mit jeweils 500 ppm Omeprazol
(V1) alleine sowie
   (1) mit 100 ppm Eriodictyol (ED) bzw.
   (2) mit 100 ppm Homoeriodictyolnatriumsalz (HE) bzw.
   (3) mit 100 ppm Eriodictyol + 100 ppm Homoeriodictyolnatriumsalz bzw.
(V2) sowie mit 100 ppm 4'-Fluor-6-methoxyflavanon als Vergleichssubstanz, die keinen Maskierungseffekt hat versetzt und verkostet. Die Lösungen 2 - 5 wurden jeweils in Duo-Vergleichstest in randomisierter Reihenfolge gegen die Lösung 1 von einem erfahrenen Panel bestehend aus 20-30 Testern verkostet und die Bitterkeit jeder Probe auf einer Skala von 0 bis 100 eingeschätzt. Die Mittelwerte der Bewertungen sind in **Tabelle 1** zusammengefasst.

**Tabelle 1**

| Biitterbewertung Omeprazol | | | |
|---|---|---|---|
| **Beisp iel** | | **Bitterkeit** | **Verminderung gegenüber Kontrollversuch** |
| V1 | Omeprazol | 80 | |
| 1 | Omeprazol + ED | 59 | -26 |
| 2 | Omeprazol + HE | 71 | -11 |
| 3 | Omeprazol + ED + HE | 44 | -45 |
| V2 | Omeprazol + 4'-Fluor-6-methoxyflavanon | 86 | +8 |

Wie man erkennen kann, wirken sowohl ED als HE allein für sich als Maskierer für die Bitterkeit von Omeprazol. Überraschend ist die Kombination der Stoffe mit -45 % deutlich besser wirksam als die zu erwartende additive Maskierung von -37 %. Die strukturell verwandte Vergleichsverbindung in Beispiel V2 ist nicht wirksam, sondern verstärkt im Gegenteil sogar die Bitterkeit von Omeprazol.

### ANWENDUNGSBEISPIELE

### Anwendungsbeispiel 1

**Sirup mit Pantoprazol und ED/HED (Mengenangaben als Gew.-%)**

| **Inhaltsstoffe** | **A** | **B** |
|---|---|---|
| Glycerol | 10.0 | 10.0 |
| Zuckersirup | 45.0 | 45.0 |
| Pantoprazol 8 % in Wasser | 10.0 | 10.0 |
| Aspartam 5% in Wasser | 5.0 | 5.0 |
| Symrise Aroma | 0.4 | - |
| Symrise Aroma mit 1.25% ED und 1.25% HED | - | 0.4 |
| Wasser | auf 100 | auf 100 |

Zubereitung eines Sirups mit 40 mg Pantoprazol/Dosis nach obiger Rezeptur. Die Ansätze werden gründlich gemischt bis alle Komponenten gelöst sind. Verglichen mit der Vergleichszubereitung A war die Zubereitung B deutlich weniger bitter.

### Anwendungsbeispiel 2

**Pulverzubereitung mit Omeprazol und ED/HED (Mengenangaben als 'Gew.-%)**

| **Inhaltsstoffe** | **A** | **B** |
|---|---|---|
| Omeprazol | 0.4 | 0.4 |
| Zitronensäure Anhydrat | 3.0 | 3.0 |
| Aspartam | 2.5 | 2.5 |
| Ascorbinsäure | 1.0 | 1.0 |
| Symrise Aroma | 0.8 | - |
| Symrise Aroma mit 0.625% ED und 0.625% HED | - | 0.8 |
| Saccharose | auf 100 | auf 100% |

Zubereitung einer Pulvermischung (5 g) mit 20 mg Omeprazol/Dosis nach obiger Rezeptur. Die beiden Ansätze werden trocken gemischt und anschließend gesiebt. Verglichen mit der Vergleichszubereitung A war die Zubereitung B deutlich weniger bitter.

### Anwendungsbeispiel 3

**Kautablette mit Omeprazol und ED/HED (Mengenangaben als Gew.-%)**

| **Inhaltsstoffe** | **A** | **B** |
|---|---|---|
| Calciumcarbonat | 25.0 | 25.0 |
| Magnesiumstearat | 0.5 | 0.5 |
| Omeprazol | 0.5 | 0.5 |
| Zitronensäure | 0.75 | 0.75 |
| Aspartam | 0.075 | 0.075 |
| Symrise Aroma | 0.8 | - |
| Symrise Aroma mit 0.625% ED und 0.625% HED | - | 0.8 |
| Dextrose | Auf 100 | Auf 100 |

Herstellung von Kautabletten (2g) mit 10 mg Omeprazol/Dosis nach obiger Rezeptur. Alle Komponenten werden gemischt und nach 1-2 h zu Tabletten gepresst. Verglichen mit der Vergleichszubereitung A war die Zubereitung B deutlich weniger bitter.

### Anwendungsbeispiel 4

**Brausetablette mit Pantoprazol und ED/HED (Mengenangaben als Gew.-%)**

| **Inhaltsstoffe** | **A** | **B** |
|---|---|---|
| Omeprazol | 2.0 | 2.0 |
| Sorbitol | 8.4 | 8.4 |
| Natriumcyclamat | 1.5 | 1.5 |
| Saccharin | 0.25 | 0.25 |
| Propylenglycol | 0.625 | 0.625 |
| Symrise Aroma | 0.8 | - |
| Symrise Aroma mit 0.625% ED und 0.625% HED | - | 0.8 |

Herstellung einer Brausetablette (2g) mit 40 mg Pantoprazol/Dosis nach obiger Rezeptur. Alle Komponenten werden gründlich gemischt und dann mit einer vorbereiteten Mischung aus Natriumhydrogencarbonat und Zitronensäure (im Gewichtsverhältnis 1:1.36) auf 100% aufgefüllt. Nach 1-2 h wird gesiebt und anschließend die Verpressung zu Tabletten vorgenommen. Verglichen mit der Vergleichszubereitung A war die Zubereitung B deutlich weniger bitter.

### Anwendungsbeispiel 5

**Fruchtgummi mit Pantoprazol und ED/HED (Mengenangaben als Gew.-%)**

| **Inhaltsstoffe** | **A** | **B** |
|---|---|---|
| Gelatine | 7.6% | 7.6% |
| Pantoprazol 8% in Wasser | 5% | 5% |
| Symrise-Aroma mit Farbstoff | 0.8% | - |
| Symrise-Aroma mit Farbstoff und 0.625% ED und 0.625% HED | - | 0.8% |
| Wasser | Auf 100% | Auf 100% |

Zur Herstellung eines Fruchtgummis mit 20 mg Pantoprazol pro Dosis werden die in der obigen Rezeptur gelisteten Inhaltsstoffe zu einer aufgekochten Zuckersirupmischung (89° Brix) zugegeben. Verglichen mit der Vergleichszubereitung A war die Zubereitung B deutlich weniger bitter.

## Patentansprüche

1. Zubereitung, enthaltend
(a) Omeprazol und/oder Pantoprazol und
(b) Eriodictyol und Homoeriodictyol.

2. Zubereitung nach Anspruch 1, **dadurch gekennzeichnet, dass** sie die Komponenten (a) und (b) im Gewichtsverhältnis von etwa 4:1 bis 400:1 enthält.

3. Zubereitung nach den Ansprüchen 1 und/oder 2, **dadurch gekennzeichnet, dass** sie als Komponente (a) Omeprazol in einer Menge von etwa 2.000 bis etwa 20.000 ppm enthält.

4. Zubereitung nach mindestens einem der Ansprüche 1 bis 3, **dadurch gekennzeichnet, dass** sie als Komponente (b) Eriodictyol und Homoeriodictyol in einer Menge von etwa 50 bis etwa 500 ppm enthält.

5. Zubereitung nach Anspruch 4, **dadurch gekennzeichnet, dass** sie als Komponente (b) Eriodictyol und Homoeriodictyol jeweils in einer Menge von etwa 25 bis etwa 250 ppm enthält.

6. Zubereitung nach mindestens einem der Ansprüche 1 bis 5, **dadurch gekennzeichnet, dass** sie in fester Form vorliegt.

7. Zubereitung nach Anspruch 6, **dadurch gekennzeichnet, dass** sie eine Tablette oder ein Kaugummi darstellt.

8. Zubereitung nach Anspruch 7, **dadurch gekennzeichnet, dass** sie ferner Trägerstoffe, Sprengmittel und/oder Farb- und Aromastoffe enthält.

9. Zubereitung nach Anspruch 6, **dadurch gekennzeichnet, dass** sie eine Kapsel darstellt.

10. Zubereitung nach mindestens einem der Ansprüche 6 bis 9, **dadurch gekennzeichnet, dass** sie einen säureresistenten Überzug oder Hülle besitzt.

11. Zubereitung nach Anspruch 1 als Arzneimittel.

12. Zubereitung nach Anspruch 1 zur Verwendung als Arzneimittel zur Behandlung von Entzündungszuständen des Magens und des Darms sowie Refluxösophagitis.

13. Arzneimittel nach den Ansprüchen 11 oder 12, **dadurch gekennzeichnet, dass** sie zur oralen Aufnahme bestimmt sind.

14. Zubereitung nach mindestens einem der Ansprüche 1 bis 10 oder Arzneimittel nach Anspruch 11 oder 12, **dadurch gekennzeichnet, dass** sie als Tabletten, Kapseln, Kautabletten, Lösungen, Emulsionen, Suspensionen, Pulver zur direkten Einnahme, Kaubonbons, Lutschbonbons, Gummibonbons, Kaugummis oder Fruchtgummis vorliegen.

15. Verfahren zur Maskierung des Bittergeschmacks von pharmazeutischen Zubereitungen mit einem Gehalt an Omeprazol oder Pantoprazol, umfassend die folgenden Schritte:
(i) Bereitstellen einer Trägermasse zur Herstellung einer pharmazeutischen Zubereitung,
(ii) Versetzen der Masse aus Schritt (i) mit einer Menge an Omeprazol oder Pantoprazol,
(iii) Versetzen der Masse aus Schritt (ii) mit einer Menge an Eriodictyol und Homoeriodictyol, und
(iv) Verarbeiten der Masse aus Schritt (iii) zu einem pharmazeutischen Endprodukt.

16. Verwendung von Eriodictyol und Homoeriodictyol zur Maskierung oder Inhibierung des Bittergeschmacks von Omeprazol oder Pantoprazol.

## Claims

1. Composition, comprising
(a) omeprazole and/or pantoprazole and
(b) eriodictyol and homoeriodictyol.

2. Composition according to claim 1, **characterized in that** the composition comprises the components (a) and (b) in a weight ratio of approximately 4:1 to 400:1.

3. Composition according to claims 1 and/or 2, **characterized in that** the composition comprises as component (a) omeprazole in an amount of approximately 2,000 to approximately 20,000 ppm.

4. Composition according to at least one of the claims 1 to 3, **characterized in that** the composition comprises as component (b) eriodictyol and homoeriodictyol in an amount of approximately 50 to approximately 500 ppm.

5. Composition according to claim 4, **characterized in that** the composition comprises as component (b) eriodictyol and homoeriodictyol each in an amount of approximately 25 to approximately 250 ppm.

6. Composition according to at least one of the claims 1 to 5, **characterized in that** the composition is present in solid form.

7. Composition according to claim 6, **characterized in that** the composition is a tablet or a chewing gum.

8. Composition according to claim 7, **characterized in that** the composition further comprises carriers, disintegrants and/or dyes and aromatic substances.

9. Composition according to claim 6, **characterized in that** the composition is a capsule.

10. Composition according to at least one of claims 6 to 9, **characterized in that** the composition has an acid-resistant coating or shell.

11. Composition according to claim 1 as pharmaceutical.

12. Composition according to claim 1 for use as pharmaceutical for treating inflammatory states of the stomach and the intestine as well as reflux esophagitis.

13. Pharmaceuticals according to claims 11 or 12, **characterized in that** the pharmaceuticals are intended for oral intake.

14. Composition according to at least one of claims 1 to 10 or pharmaceutical according to claim 11 or 12, **characterized in that** the composition or the pharmaceutical are present as tablets, capsules, chewable tablets, solutions, emulsions, suspensions, powders for direct intake, soft candies, hard candies, gumdrops, chewing gums or fruit gums.

15. Method for masking the bitter taste of pharmaceutical compositions with an amount of omeprazole or pantoprazole, comprising the following steps:
(i) Providing a carrier mass for producing a pharmaceutical composition,
(ii) Adding to the mass of step (i) an amount of omeprazole or pantoprazole,
(iii) Adding to the mass of step (ii) an amount of eriodictyol and homoeriodictyol, and
(iv) Processing the mass of step (iii) to a pharmaceutical finished product.

16. Use of eriodictyol and homoeriodictyol for masking or inhibiting the bitter taste of omeprazole or pantoprazole.

## Revendications

1. Préparation contenant
(a) de l'oméprazole et/ou du pantoprazole et
(b) de l'ériodictyol et de l'homoériodictyol.

2. Préparation selon la revendication 1, **caractérisée par le fait qu'**elle contient les composants (a) et (b) dans un rapport pondéral compris entre environ 4 : 1 et 400 : 1.

3. Préparation selon les revendications 1 et/ou 2, **caractérisée par le fait qu'**elle contient en tant que composant (a) de l'oméprazole en une quantité comprise entre environ 2.000 et environ 20.000 ppm.

4. Préparation selon au moins l'une quelconque des revendications 1 à 3, **caractérisée par le fait qu'**elle contient en tant que composant (b) de l'ériodictyol et de l'homoériodictyol en une quantité comprise entre environ 50 et environ 500 ppm.

5. Préparation selon la revendication 4, **caractérisée par le fait qu'**elle contient en tant que composant (b) de l'ériodictyol et de l'homoériodictyol chacun en une quantité comprise entre environ 25 et environ 250 ppm.

6. Préparation selon au moins l'une quelconque des revendications 1 à 5, **caractérisée par le fait qu'**elle se présente sous forme solide.

7. Préparation selon la revendication 6, **caractérisée par le fait qu'**elle constitue un comprimé ou un chewing-gum.

8. Préparation selon la revendication 7, **caractérisée par le fait qu'**elle contient en outre des excipients, des désintégrants et/ou des colorants et des substances aromatisantes.

9. Préparation selon la revendication 6, **caractérisée par le fait qu'**elle constitue une capsule.

10. Préparation selon au moins l'une quelconque des revendications 6 à 9, **caractérisée par le fait qu'**elle présente un revêtement ou une enveloppe résistante(e) aux acides.

11. Préparation selon la revendication 1 en tant que médicament.

12. Préparation selon la revendication 1 destinée à être utilisée en tant que médicament pour le traitement des états inflammatoires de l'estomac et de l'intestin ainsi que de l'œsophagite par reflux.

13. Médicaments selon les revendications 11 ou 12, **caractérisés par le fait qu'**ils sont destinés à la prise orale.

14. Préparation selon au moins l'une quelconque des revendications 1 à 10 ou médicament selon la revendication 11 ou 12, **caractérisés par le fait qu'**ils se présentent en tant que comprimés, gélules, comprimés à croquer, solutions, émulsions, suspensions, poudres à ingestion directe, bonbons à mâcher, pastilles, gouttes de gomme, chewing-gums ou gommes aux fruits.

15. Procédé pour masquer le goût amer de préparations pharmaceutiques ayant une teneur en oméprazole ou en pantoprazole, comprenant les étapes suivantes consistant à:
(i) fournir une masse support pour la production d'une préparation pharmaceutique,
(ii) ajouter une quantité d'oméprazole ou de pantoprazole à la masse de l'étape (i),
(iii) ajouter une quantité d'ériodictyol et d'homoériodictyol à la masse de l'étape (ii), et
(iv) traiter la masse de l'étape (iii) pour obtenir un produit final pharmaceutique.

16. Utilisation d'ériodictyol et d'homoériodictyol pour masquer ou inhiber le goût amer d'oméprazole ou de pantoprazole.
